(19) Europäisches Patentamt
European Patent Office
Office européen des brevets

(11) **EP 3 581 090 A1**

(12) **EUROPEAN PATENT APPLICATION**

(43) Date of publication:
18.12.2019 Bulletin 2019/51

(51) Int Cl.:
*A61B 5/00* (2006.01)    *A61B 5/04* (2006.01)

(21) Application number: 18176929.0

(22) Date of filing: 11.06.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **HELLE, Michael Günter**
**5656 AE Eindhoven (NL)**
• **KATSCHER, Ulrich**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **ELECTRICAL PROPERTIES TOMOGRAPHY MAPPING OF CONDUCTIVITY CHANGES**

(57)    The invention provides for a medical imaging system (100, 300) comprising: a memory (110) for storing machine executable instructions (120); and a processor (104) for controlling the medical imaging system. Execution of the machine executable instructions causes the processor to: receive (200) a resting group of B1 phase maps (122) of a region (309) of interest of a subject (318); receive (202) an active group of B1 phase maps (124) of the region of interest of the subject; calculate (204) a resting group of conductivity maps (126) for the region of interest using the resting group of B1 phase maps according to an electrical properties tomography algorithm; calculate (206) an active group of conductivity maps (128) for the region of interest using the active group of B1 phase maps according to the electrical properties tomography algorithm; and calculate (208) a conductivity change mapping (130) for the region of interest using the resting group of conductivity maps and the active group of conductivity maps.

Fig. 3

**Description**

FIELD OF THE INVENTION

The invention relates to magnetic resonance imaging, in particular to electrical properties tomography.

BACKGROUND OF THE INVENTION

Magnetic resonance imaging (MRI) scanners rely on a large static magnetic field (B0) to align the nuclear spins of atoms as part of the procedure for producing images within the body of a patient. These images can reflect various quantities or properties of the subject. For example, the hemodynamic response of brain activation causes magnetic and electric changes in the activated brain area. MRI allows visualizing magnetic changes, e.g. based on the cerebral blood flow or blood-oxygen-level dependent (BOLD) effect. The latter is usually referred to as functional MRI (fMRI).

The review article Katscher and van den Berg, "Electric properties tomography: Biochemical, physical and technical background, evaluation and clinical applications," NMR in Biomedicine 2017;e:2729 (DOI: 10.1002/nmb.3729) discloses forward based and others methods of electrical properties tomography (EPT).

SUMMARY OF THE INVENTION

The invention provides for a medical imaging system, a computer program product, and a method in the independent claims. Embodiments are given in the dependent claims.

Embodiments may provide for the use of Electric Properties Tomography (EPT) to measure electric conductivity and/or permittivity during brain activation or changes in brain activity. The measurements are performed similar to conventional fMRI experiments, whereas EPT maps are generated for activation and resting periods. The data can be subsequently analyzed by using statistical methods (such as the t-test), but it is also possible to quantify electrical properties during activation and resting periods.

In one aspect the invention provides for a medical imaging system that comprises a memory for storing machine-executable instructions. The medical imaging system further comprises a processor for controlling the medical imaging system. Execution of the machine-executable instructions causes the processor to receive a resting group of B1 phase maps of a region of interest of a subject. The term 'resting group of B1 phase maps' as used herein is a group of B1 phase maps. The term 'resting' is to indicate a particular group of B1 phase maps.

Execution of the machine-executable instructions further causes the processor to receive an active group of B1 phase maps of the region of interest of the subject. The term 'active group of B1 phase maps' as used herein encompasses a group of B1 phase maps. The 'active' before the 'B1 phase maps' is intended to indicate a particular group of B1 phase maps. Execution of the machine-executable instructions further causes the processor to calculate a resting group of conductivity maps for the region of interest using the resting group of B1 phase maps according to an electrical properties tomography algorithm. Again, the term 'resting' in the resting group of conductivity maps is for indicating a particular group of conductivity maps. Execution of the machine-executable instructions further causes the processor to calculate an active group of conductivity maps for the region of interest using the active group of B1 phase maps according to the electrical properties tomography algorithm. The term 'active group of conductivity maps' as used herein is a group of conductivity maps and the term 'active' before is intended to indicate a particular group of conductivity maps.

Execution of the machine-executable instructions further cause the processor to calculate a conductivity change mapping for the region of interest using the resting group of conductivity maps and the active group of conductivity maps. This embodiment may be beneficial because it may provide for an effective means of producing a mapping that shows a change in conductivity for two different states of the subject. The calculation of the conductivity change mapping may be affected in different means in different examples. For example, in one embodiment the resting group of conductivity maps could be used to calculate an average resting group of conductivity maps and the active group of conductivity maps could be used to calculate an average active conductivity map.

The conductivity change mapping could then be the difference between these two average conductivity maps. In other examples the two groups may be used and various statistical methods may be applied. For example, in functional magnetic resonance imaging there are a number of techniques which use repeated measurements to determine the location of functional activity in for example the brain. These techniques could be directly applied to the resting group of conductivity maps and the active group of conductivity maps.

The resting group of B1 phase maps may for example be a three-dimensional dataset, a set of two-dimensional slices or even, in some examples, simply a single two-dimensional slice each for the region of interest.

In another embodiment the region of interest of the subject is a brain region of interest of the subject.

In another embodiment the resting group of B1 phase maps image a brain. The active group of B1 phase maps image the brain. The conductivity change mapping is descriptive of a difference in brain activity. This embodiment may be beneficial because it provides for an alternative means of functional imaging for the brain. The change in conductivity may be related to a change in the flow of blood to different regions of the brain.

In an example of this embodiment, a subject whose brain is being imaged performs an activity when the active group of B1 phase maps is acquired and the subject refrains from performing this activity when the resting group of B1 phase maps is acquired. The conductivity change mapping may then indicate increases in blood flow to a particular part of the brain caused by performing the activity. For example, the subject could move a particular body part such as a hand when the active group of B1 phase maps is acquired. In another example the subject could think particular thoughts or receive a stimulus such as light or sound when the active group of B1 phase maps is acquired.

In another embodiment the electrical properties tomography algorithm of the calculation of the active group of conductivity maps and the resting group of conductivity maps is at least partially implemented as a machine learning algorithm. For example, the differential equations may be solved using a neural network.

In another embodiment the electrical properties tomography algorithm of the calculation of the active group of conductivity maps and the resting group of conductivity maps is at least partially implemented as a forward differential equation solver. This embodiment may be beneficial because it provides for a numerically efficient means of calculating the various conductivity maps.

In another embodiment the forward differential equation solver is a finite difference algorithm.

In another embodiment the forward differential equation solver is configured to calculate the conductivity of each voxel using the Laplacian of the B1 phase map for a kernel of voxels surrounding each voxel.

In another embodiment execution of the machine-executable instructions further causes the processor to receive a tissue segmentation assigning each voxel in the region of interest a tissue type.

Execution of the machine-executable instructions further causes the processor to adjust the kernel of voxels surrounding each voxel using the tissue segmentation before calculating Laplacian. The kernel of voxels surrounding each voxel is adjusted such that the voxels within each kernel have the same tissue type. This embodiment may be beneficial because it provides for a numerically accurate means of calculating the conductivity maps.

In another embodiment execution of the machine-executable instructions further causes the processor to receive a magnetic resonance image descriptive of the region of interest. The magnetic resonance image may for example be a magnitude image. Execution of the machine-executable instructions further causes the processor to segment the magnetic resonance image to generate the tissue segmentation that assigns each voxel in the region of interest a tissue type.

In another embodiment execution of the machine-executable instructions further causes the processor to receive a magnitude image of the region of interest. Execution of the machine-executable instructions further causes the processor to render the magnitude image and conductivity change mapping on a display. This may be affected in several different ways. In one way the conductivity change mapping is superimposed on the magnitude image. In another way the conductivity change mapping and the magnitude image are displayed in adjacent regions with an identical scale. Both of these may provide for a means which enables a physician or operator to easily interpret the results of the conductivity change mapping.

The magnitude image in this example may take different forms. It could be a separately acquired proton density image or other magnetic resonance image, it could also be an average of images acquired when acquiring the B1 phase maps.

In another embodiment execution of the machine-executable instructions further causes the processor to receive magnetic resonance imaging data acquired according to a B1 phase mapping magnetic resonance imaging protocol descriptive of the region of interest of the subject. Execution of the machine-executable instructions further causes the processor to receive meta data that assigns portions of magnetic resonance imaging data to a resting state of the subject or an active state of the subject.

Execution of the machine-executable instructions further causes the processor to reconstruct multiple B1 magnetic resonance phase maps of the region of interest of the subject from the portions of the magnetic resonance imaging data. Execution of the machine-executable instructions further causes the processor to construct the active group of B 1 phase maps and the resting group of B1 phase maps by assigning each of the multiple B 1 magnetic resonance phase maps using the meta data. In this embodiment the raw magnetic resonance imaging data is reconstructed into B1 magnetic resonance phase maps and then they are assigned to either the active group or the resting group using the meta data.

In another embodiment the medical imaging system further comprises a magnetic resonance imaging system configured for acquiring the magnetic resonance imaging data from the subject from an imaging zone. The memory further comprises pulse sequence commands. These pulse sequence commands are configured to control the magnetic resonance imaging system to acquire the magnetic resonance imaging data from the region of interest according to the B1 phase mapping magnetic resonance imaging protocol. The region of interest is within the imaging zone. Execution of the machine-executable instructions further causes the processor to control the magnetic resonance imaging system to acquire the magnetic resonance imaging data using the pulse sequence commands.

In another embodiment the B 1 phase mapping magnetic resonance imaging protocol is any one of the following: a balanced steady state free precession magnetic resonance imaging protocol, a multi-echo gradient echo magnetic resonance imaging protocol, and a spin-echo based magnetic resonance imaging protocol.

In another embodiment the magnetic resonance imaging system further comprises a subject indicator configured for indicating the resting state and the active state to the subject. Execution of the machine-executable instructions further causes the processor to control the magnetic resonance imaging system to repeatedly acquire the magnetic resonance imaging data while the subject indicator alternates between the resting state and the active state. In these for each of the acquisitions the acquisition is entirely within one of the resting state and the acting active state. Execution of the machine-executable instructions further causes the processor to generate the meta data for the magnetic resonance imaging data to match the subject indicator during the acquisition of the magnetic resonance imaging data.

In another embodiment the resting group of B1 phase maps and the active group of B1 phase maps each contain any one of the following: at least 5 B1 phase maps each, at least 10 B1 phase maps each, at least 20 B 1 phase maps each, at least 40 B 1 phase maps each, at least 60 B1 phase maps each, and at least 80 B 1 phase maps each.

In other embodiments various B1 magnitude images may be acquired and permittivity mapping may also be performed in conjunction with the conductivity mapping. In some embodiments, a change in the permittivity may also be displayed with the change in the conductivity mapping.

In another aspect the invention provides for a method of operating a medical imaging system. The method comprises receiving a resting group of B 1 phase maps from a region of interest of the subject. The method further comprises receiving an active group of B1 phase maps of the region of interest of the subject. The method further comprises calculating a resting group of conductivity maps for the region of interest using the resting group of B 1 phase maps according to an electrical properties tomography algorithm. The method further comprises calculating an active group of conductivity maps for the region of interest using the active group of B1 phase maps according to the electrical properties tomography algorithm. The method further comprises calculating a conductivity change mapping for the region of interest using the resting group of conductivity maps and the active group of conductivity maps.

In another aspect the invention further provides for a computer program product comprising machine executable instructions for execution by a processor controlling the medical imaging system. Execution of the machine-executable instructions causes the processor to receive a resting group of B1 phase maps of a region of interest of a subject. Execution of the machine-executable instructions further causes the processor to receive an active group of B1 phase maps for the region of interest of the subject. Execution of the machine-executable instructions further causes the processor to calculate a resting group of conductivity maps for the region of interest using the resting group of B1 phase maps according to an electrical properties tomography algorithm.

Execution of the machine-executable instructions further causes the processor to calculate an active group of conductivity maps for the region of interest using the active group of B1 phase maps according to the electrical properties tomography algorithm. Execution of the machine-executable instructions further causes the processor to calculate a conductivity change mapping for the region of interest using the resting group of conductivity maps and the active group of conductivity maps.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit,' 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, random access memory (RAM), read only memory (ROM), an optical disk, a magneto-optical disk, and the register file of the processor. Examples of optical disks include compact disks (CD) and digital versatile disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising a 'processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as C or similar programming languages and compiled into machine executable instructions. In some instances, the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the internet using an internet service provider).

Aspects of the present invention are described with reference to flowchart illustrations and / or block diagrams of methods, apparatus (systems) and computer program products according to embodiments of the invention. It is understood that each block or a portion of the blocks of the flowchart, illustrations, and / or block diagrams, can be implemented by computer program instructions in form of computer executable code when applicable. It is further understood that, when not mutually exclusive, combinations of blocks in different flowcharts, illustrations, and / or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions / acts specified in the flowchart and / or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function / act specified in the flowchart and / or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions / acts specified in the flowchart and / or block diagram block or blocks.

A 'user interface' as used herein is an interface which allows a user or operator to interact with a computer or computer system. A 'user interface' may also be referred to as a 'human interface device.' A user interface may provide information or data to the operator and / or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and / or control an external computing device and / or apparatus. A hardware interface may allow a processor to send control signals or instructions to an external computing device and / or apparatus. A hardware interface may also enable a processor to exchange data with an external computing device and / or apparatus. Examples of a hardware interface include, but are not limited to: a universal serial bus, IEEE 1394 port, parallel port, IEEE 1284 port, serial port, RS-232 port, IEEE 488 port, Bluetooth connection, wireless local area network connection, TCP / IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, a tactile electronic display, a Braille screen, a cathode ray tube (CRT), a storage tube, a bi-stable display, an electronic paper, a vector display, a flat panel display, a vacuum fluorescent display (VF), light-emitting diode (LED) displays, an electroluminescent display (ELD), plasma display panels (PDP), a liquid crystal display (LCD), organic light-emitting diode displays (OLED), a projector, and a head-mounted display.

Magnetic Resonance imaging data is defined herein as being the recorded measurements of radio frequency signals emitted by atomic spins using the antenna of a magnetic resonance apparatus during an MRI scan. Magnetic resonance image data is defined herein as being the reconstructed two-dimensional or three-dimensional visualization of anatomic data that is reconstructed from the magnetic resonance k-space data. Visualization of the magnetic resonance image data can be performed using a computer.

BRIEF DESCRIPTION OF THE DRAWINGS

In the following preferred embodiments of the invention will be described, by way of example only, and with reference to the drawings in which:

Fig. 1 illustrates an example of a medical imaging system;
Fig. 2 shows a flow chart which illustrates a method of operating the medical imaging system of Fig. 1;
Fig. 3 illustrates a further example of a medical imaging system;
Fig. 4 shows a flow chart which illustrates a method of operating the medical imaging system of Fig. 3;
Fig. 5 illustrates how the multiple B1 magnetic resonance phase maps can be sorted; and
Fig. 6 illustrates examples of conductivity change mappings constructed according to some examples herein.

DETAILED DESCRIPTION OF THE EMBODIMENTS

Like numbered elements in these figures are either equivalent elements or perform the same function. Elements which have been discussed previously will not necessarily be discussed in later figures if the function is equivalent.

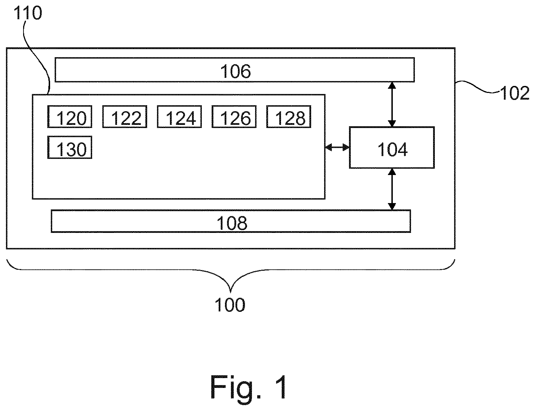

Fig. 1 illustrates an example of a medical imaging system 100. The medical imaging system 100 is shown as comprising a computer 102 that comprises a processor 104. The processor is shown as being connected to an optional hardware interface 106, and an optional user interface 108. The user interface 108 may be or include a display for rendering images. The hardware interface 106 may for example be a network interface or it may also be used for exchanging data or commands with other components of the medical imaging system. The processor 104 is further shown as being connected to a memory 110. The memory 110 may be any combination of memory which is accessible to the processor 104. This may include such things as main memory, cached memory, and also non-volatile memory such as flash RAM, hard drives, or other storage devices. In some examples the memory 104 may be considered to be a non-transitory computer-readable medium.

The memory is shown as containing machine-executable instructions 120. The machine-executable instructions 120 enable the processor 104 to perform various data processing tasks and also in some examples to control other components of the medical imaging system 100.

The memory 110 is shown as containing a resting group of B1 phase maps 122. The resting group of B1 phase maps 122 is a group of B1 phase maps that is labeled the resting group of B1 phase maps. The memory 110 is further shown as containing an active group of B1 phase maps 124. Likewise, the active group of B1 phase maps 124 is a group of B1 phase maps that is labeled active group of B1 phase maps 124. The memory 110 is further shown as containing a resting group of conductivity maps 126 that was calculated using each of the resting group of B1 phase maps 122. The memory 110 is further shown as containing an active group of conductivity maps 128 that was calculated using each of the active group of B1 phase maps 124. The memory 110 is further shown as containing a conductivity change mapping 130. The conductivity change mapping 130 is a change in conductivity for a region of interest of a subject that was calculated using the resting group of conductivity maps 126 and the active group of conductivity maps 128.

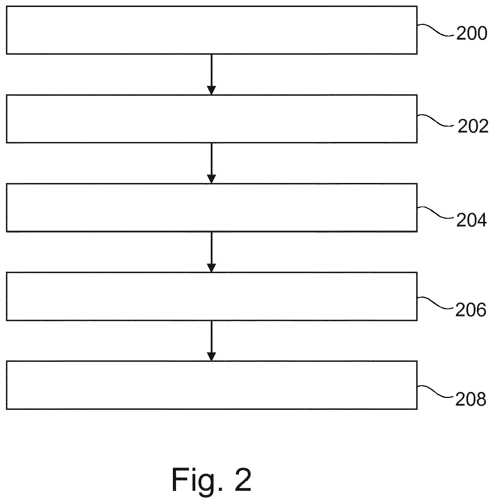

Fig. 2 shows a flowchart which illustrates a method of operating the medical imaging system 100 of Fig. 1. First in step 200 the processor 104 receives the resting group of B1 phase maps 122 which are for a region of interest of a subject. Next in step 202 the processor 104 receives the active group of B1 phase maps 124 of the region of interest of the subject. The resting group of B1 phase maps 122 and the active group of B1 phase maps 124 could be received in different ways. In one case they may be received via a network or other data transfer method. In other examples the medical imaging system could be part of a magnetic resonance imaging system and the resting group of B1 phase maps 122 and the active group of B1 phase maps 124 could be received by reconstructing them from magnetic resonance imaging data.

Next in step 204 the resting group of conductivity maps 126 is calculated for the region of interest from the resting group of B1 phase maps. Next in step 206 the active group of conductivity maps 128 is calculated for the region of interest using the active group of B1 phase maps 124. The resting group of conductivity maps 126 and the active group of conductivity maps 128 are both calculated according to an electrical properties tomography algorithm. Finally in step 208 the conductivity change mapping 130 is calculated for the region of interest using the resting group of conductivity maps 126 and the active group of conductivity maps 128. Optional steps which are not displayed in Fig. 2 may include such things as rendering the conductivity change mapping which may also include rendering the conductivity change mapping on a display with a magnetic resonance image that illustrates the region of interest.

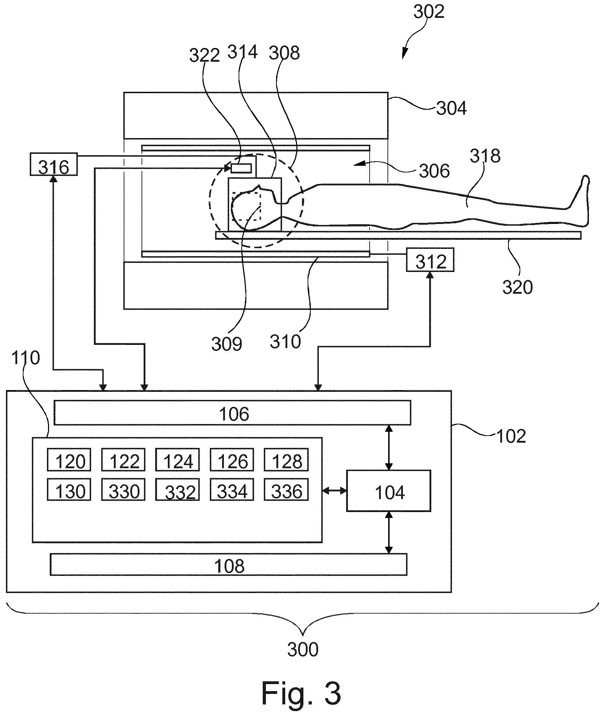

Fig. 3 illustrates a further example of a medical imaging system 300. The medical imaging system 300 in Fig. 3 is similar to the medical imaging system 100 of Fig. 1 with the exception that the medical imaging system 300 also comprises a magnetic resonance imaging system.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquired for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. In this example the radio-frequency coil 314 is a head coil and the region of interest 309 images the brain of the subject 318.

The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receiver. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

Within the bore 306 of the magnet 304 there is a subject indicator 322. The subject indicator may for example provide an audio and/or visual stimulus to the subject 318. The subject indicator 322 is able to provide a stimulus in one of two different distinct states; an active state and a resting state. When the subject indicator 322 shows an active state the subject 318 either thinks particular thoughts or performs particular physical activity such as moving a limb or performing another action. The subject indicator 322 could for example have a light which is visible to the subject 318, be a display, or provide an audio signal. The transceiver 316, the gradient controller 312, and the subject indicator 322 are shown as being connected to the hardware interface 106 of the computer system 102.

The memory 110 is further shown as containing the pulse sequence commands 330. The pulse sequence commands are either commands or data which can be converted into such commands which enable the processor 104 to control the magnetic resonance imaging system 302. The memory 110 is further shown as containing magnetic resonance imaging data 332 that was acquired by controlling the magnetic resonance imaging system 302 with the pulse sequence commands 330. The pulse sequence commands 330 may also contain instructions which cause the subject indicator 322 to change between indicating the active and resting state during individual acquisitions of the magnetic resonance imaging data 332. Data which can be used to determine later which state the magnetic resonance imaging data 332 is in the meta data 334.

The meta data 334 is shown as being stored in the memory 110. The memory 110 shows multiple B1 magnetic resonance phase maps 336 that have been reconstructed from the magnetic resonance imaging data 332. The meta data 334 is a key which can be used to determine which of the multiple B1 magnetic resonance phase maps belong to the resting group of B1 phase maps 122 and the active group of B1 phase maps 124. Using the meta data 334 the processor 104 can sort the multiple B1 magnetic resonance phase maps 336 into the resting group of B1 phase maps 122 and the active group of B1 phase maps 124.

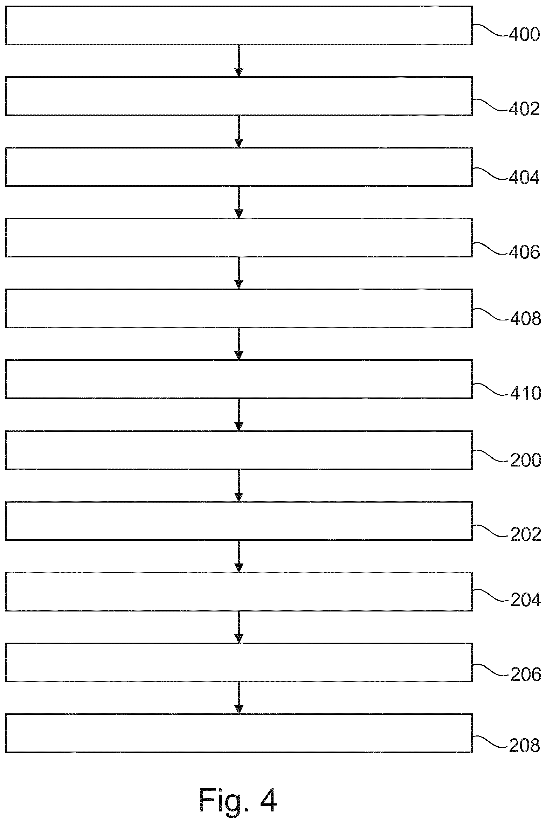

Fig. 4 shows a flowchart which illustrates a method of operating the medical imaging system 300 of Fig. 3. The method in Fig. 4 is similar to the method illustrated in Fig. 2 with the addition of a number of additional steps. The method of Fig. 4 starts with step 400. In step 400 the processor 104 controls the magnetic resonance imaging system 302 to repeatedly acquire the magnetic resonance imaging data 332. As step 400 is performed, step 402 is also performed. In step 402 the processor 104 generates the meta data 334 for the magnetic resonance imaging data 332. This is so that the meta data matches the subject indicator 322 during acquisition of the magnetic resonance imaging data.

Next in step 404 the processor receives the magnetic resonance imaging data 332. The magnetic resonance imaging data for this method has been acquired for the region of interest 309 of the subject's 318 brain region. Next in step 406 the processor 104 receives the meta data 334. Next in step 408 the processor reconstructs the multiple B1 magnetic resonance phase maps 336 from the portions of the magnetic resonance imaging data 332. Next in step 410 the processor 104 constructs or sorts the active group of B1 phase maps 124 and the resting group of phase maps 122 by assigning them each of the multiple B1 magnetic resonance phase maps using the meta data 334. After step 410 the method then proceeds to step 200 as is illustrated in Fig. 2.

Magnetic resonance imaging is a very valuable tool to visualize anatomy and morphology of different organs of the body. In addition to that, MRI can be used to measure brain activity by detecting changes associated with cerebral blood flow, also referred to as functional magnetic resonance imaging (fMRI). It relies on the fact that cerebral blood flow and neuronal activation are coupled. When a certain area of the brain is in use (e.g. parts of the motor cortex when a person is moving muscles willingly) the cerebral blood flow to that region as well as the oxygen consumption increases.

The hemodynamic response of brain activation causes magnetic and electric changes in the activated brain area. Up to now, it is only known how to image the magnetic changes of the hemodynamic response with MRI, e.g. based on cerebral blood flow (CBF) changes or magnetic properties of deoxygenated blood. It is not known how to image the electric changes of the hemodynamic response.

The primary form of fMRI is based on the blood-oxygen-level dependent (BOLD) effect. Hemoglobin presents different magnetic properties in its oxygenated and deoxygenated forms which lead to magnetic signal variation that can be detected using an MRI scanner, usually by a T2*-sensitive sequence. Given many repetitions of an action performed by a subject while scanning, statistical methods can be used to determine the areas of the brain which reliably have more of this difference as a result, and therefore which areas of the brain are most active during that action. The resulting brain activation can be color-coded and superimposed to previously acquired anatomical images, thus, visualizing active parts of the brain.

fMRI is clinically used to visualize brain activation with respect to a tumor in order to enable a surgeon to plan a surgery or make other therapy decisions. In neuro science, fMRI is a welcome tool to study complex processes in the brain related to behavior, action etc. which may lead to a better understanding of various diseases like depression, schizophrenia, autism, epilepsy etc.

Examples may provide for the use of Electric Properties Tomography (EPT) to measure electric conductivity and/or permittivity during brain activation. The measurements are performed similar to conventional fMRI experiments, but instead of the BOLD contrast EPT maps are generated for activation and resting periods. The data can be subsequently analysed by using statistical methods, but it is also possible to quantify electrical properties during activation and resting periods. BOLD measurements mainly rely on alterations due to deoxygenated blood increase, so that the signal may be biased towards the venous blood which would not be the case for the proposed approach.

The increase in cerebral blood flow increases the conductivity in the activated area (since blood conductivity ~ 1.25 S/m is higher than gray/white matter conductivity ~0.45 S/m) as well as the permittivity in the activated area (since (relative) blood permittivity ~ 70 is higher than (relative) gray/white matter permittivity ~60).

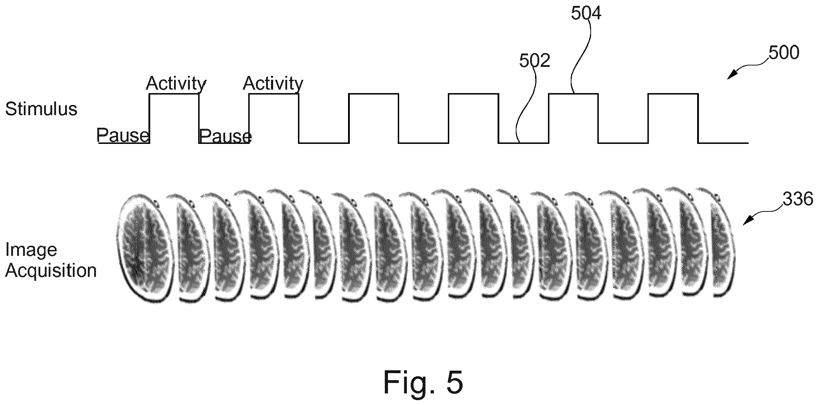

An EPT fMRI measurement can be performed using a balanced steady-state free precession (bSSFP) sequence (pulse sequence commands 330) for data acquisition. The image volume of the scan covers the whole brain or a single region where activity is expected (region of interest 309). The image volume is acquired several times, e.g. 60 dynamics are performed. During scanning, a volunteer or patient is being advised to perform a certain task alternating with resting periods, e.g. finger tapping, looking at pictures, producing words etc. (cf Fig. 5 below). In addition to the reconstructed magnitude images also the phase images of the scans are stored for subsequent reconstruction of electrical conductivity maps via

$$\sigma = \Delta\varphi/(2\mu\omega^2), \tag{1}$$

where $\omega$ = Larmor frequency, $\Delta$ = Laplacian operator (second spatial derivative in 3D), $\varphi$ = bSSFP phase map, and $\mu$ = magnetic permeability of the body. Conductivity is reconstructed for all 60 dynamics separately using Eq. (1), and results are investigated using the statistical methods usually applied for fMRI. The same procedure can be done reconstructing tissue permittivity, which is enabled if additionally the brain B1 map (i.e., the magnitude of the RF transmit field) is measured. Conductivity-based fMRI is more promising than permittivity-based fMRI, since (a) phase can be measured faster and more accurate than B1 magnitude, (b) differences between blood and brain are higher for conductivity than permittivity.

Comparing conductivity maps from the different dynamics, any errors from imperfect measurement or imperfect reconstruction cancel out, as long as these errors are (roughly) the same for all dynamics.

Fig. 5 shows a Fig. which illustrates how the multiple B1 magnetic resonance phase maps 336 can be sorted. The patient (subject 318) is being advised to perform a certain task (by the subject indicator 322) while the image volume is constantly being acquired. The top part of the plot shows a square wave waveform that indicates the signal 500 provided by the subject indicator 322. The two states have an activity state 504 and a resting state or inactivity 502. Below this is a series of images which represent the multiple B1 magnetic resonance phase maps 336. The data from the waveform 500 can be correlated to each of the multiple B1 magnetic resonance phase maps 336 in the form of meta data which are then used to sort the multiple B1 magnetic resonance phase maps 336 into the resting group of B1 phase maps 122 and the active group of B1 phase maps 124.

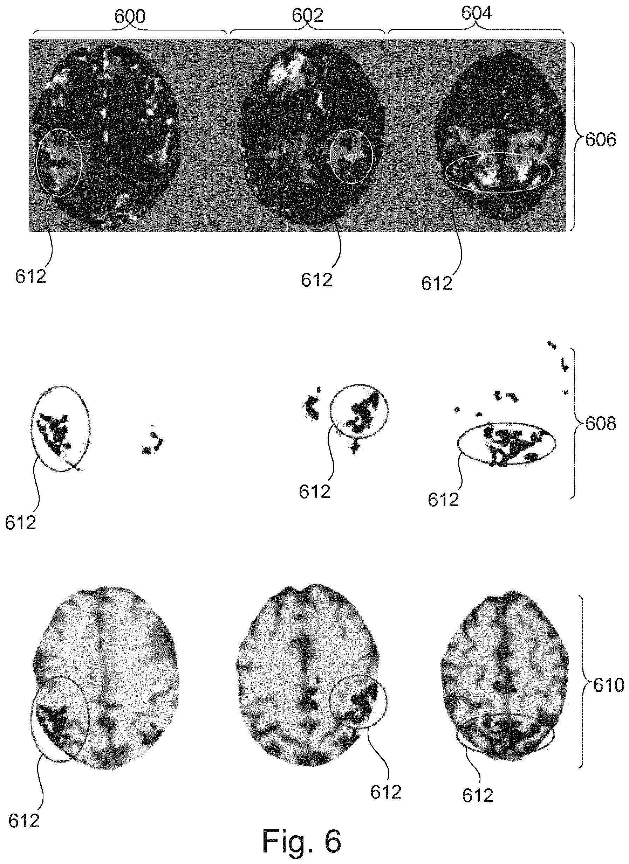

Fig. 6 illustrates examples of conductivity change mappings constructed according to some examples herein. There are nine images arranged in a matrix. In columns this corresponds to data that was acquired at identical times and for different motions of the subject. The images in column 600 correspond to left hand motion by the subject in the active state. Images in the middle column labeled 602 correspond to motion by the subject of the right hand during the active phase. The column to the far right labeled 604 corresponds to motion of the subject of both feet.

The images in the first row 606 show the results of calculating the t value between the conductivity maps with and without activation. The conductivity maps with activation correspond to the active group of conductivity maps 128. The conductivity maps without activation correspond to the resting group of conductivity maps 126. The Figs. in row 606 is one example of a conductivity change mapping 130 with and without activation. Row 608 illustrates results of a statistical t-test from a corresponding conventional fMRI study using the BOLD effect. The bottom row, row 610 shows the results of row 608 superimposed on a bSSFP magnitude image. In each column 600, 602, 604 there are regions of interest 612. Within each column the region of interest 612 is identical.

Fig. 6 above shows results for three different volunteer experiments using a 3T magnet, testing motion of the left hand, the right hand, and the feet. The area of brain activation is almost the same for fMRI using conventional EPI and using EPT as proposed. The difference in conductivity with/without activation is of the order of 0.1 S/m, corresponding to a blood volume change of 10%, which is in line with expectations. Using EPT, the activated region appears blurred towards the inner part of the brain, which can be explained by the following issue:
Solving the Laplacian of Eq. (1) numerically requires an ensemble of voxels (the so-called "kernel") around the target voxel. This kernel has to be based on voxels with the same conductivity as the target voxel to avoid reconstruction errors. Usually, this is realized by taking tissue boundaries from the bSSFP magnitude image into account, to match local geometric shape of kernel and tissue. In the case of fMRI, no tissue boundary between activated / non-activated areas was available, which is the reason for the above-mentioned observation that the activated region appears blurred. The blurring appears only towards the inner part of the brain (since no clear boundary is given on this side of the activation area), but blurring does not appear towards the outer part of the brain (since a clear boundary is given on this side of the activation area).

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 100 | medical imaging system |
| 102 | computer |
| 104 | processor |
| 106 | hardware interface |
| 108 | user interface |
| 110 | computer memory |
| 120 | machine executable instructions |
| 122 | resting group of B1 phase maps |
| 124 | active group of B1 phase maps |
| 126 | resting group of conductivity maps |
| 128 | active group of conductivity maps |
| 130 | conductivity change mapping |
| 200 | receive a resting group of B1 phase maps of a region of interest of a subject |
| 202 | receive an active group of B1 phase maps of the region of interest of the subject |
| 204 | calculate a resting group of conductivity maps for the region of interest using the resting group of B1 phase maps according to an electrical properties tomography algorithm |
| 206 | calculate an active group of conductivity maps for the region of interest using the active group of B1 phase maps according to the electrical properties tomography algorithm |
| 208 | calculate a conductivity change mapping for the region of interest using the resting group of conductivity maps and the active group of conductivity maps |
| 300 | medical imaging system |
| 302 | magnetic resonance imaging system |
| 304 | magnet |
| 306 | bore of magnet |
| 308 | imaging zone |
| 309 | region of interest |
| 310 | magnetic field gradient coils |
| 312 | magnetic field gradient coil power supply |
| 314 | radio-frequency coil |
| 316 | transceiver |
| 318 | subject |
| 320 | subject support |
| 322 | subject indicator |
| 330 | pulse sequence commands |
| 332 | magnetic resonance imaging data |
| 334 | metadata |
| 336 | multiple B1 magnetic resonance phase maps |
| 400 | control the magnetic resonance imaging system to repeatedly acquire the magnetic resonance imaging data while the subject indicator alternates between the resting state and the active state |
| 402 | generate the metadata for the magnetic resonance imaging data to match the subject indicator during the acquisition of the magnetic resonance imaging data |
| 404 | receive magnetic resonance imaging data acquired according to a B1 phase mapping magnetic resonance imaging protocol descriptive of the region of interest of the subject |
| 406 | receive metadata that assigns portions of the magnetic resonance imaging data to a resting state of the subject |

or an active state of the subject

408    reconstruct multiple B1 magnetic resonance phase maps of the region of interest of the subject from the portions of the magnetic resonance imaging data

410    construct the active group of B1 phase maps and the resting group of B1 phase maps by assigning each of the multiple B1 magnetic resonance phase maps using the metadata

500    signal provided by subject indicator

502    resting state

504    active state

600    left hand motion during active period

602    right hand motion during active period

604    motion of both feet during active period

606    t-value between conductivity maps with/without activation

608    results of conventional fMRI using BOLD

610    figures from 608 overlaid on bSSFP magnitude maps

612    region of interest with mapping

**Claims**

1. A medical imaging system (100, 300) comprising:

   - a memory (110) for storing machine executable instructions (120); and
   - a processor (104) for controlling the medical imaging system, wherein execution of the machine executable instructions causes the processor to:

      - receive (200) a resting group of B1 phase maps (122) of a region (309) of interest of a subject (318);
      - receive (202) an active group of B1 phase maps (124) of the region of interest of the subject;
      - calculate (204) a resting group of conductivity maps (126) for the region of interest using the resting group of B1 phase maps according to an electrical properties tomography algorithm;
      - calculate (206) an active group of conductivity maps (128) for the region of interest using the active group of B1 phase maps according to the electrical properties tomography algorithm; and
      - calculate (208) a conductivity change mapping (130) for the region of interest using the resting group of conductivity maps and the active group of conductivity maps.

2. The medical imaging system of claim 1, wherein the resting group of B1 phase maps image a brain, wherein the active group of B1 phase maps image the brain, and wherein the conductivity change mapping is descriptive of difference in brain activity.

3. The medical imaging system of claim 2, wherein the electrical properties tomography algorithm of the calculation of the active group of conductivity maps and the resting group of conductivity maps is at least partially implemented as a machine learning algorithm.

4. The medical imaging system of claim 2 or 3, wherein the electrical properties tomography algorithm of the calculation of the active group of conductivity maps and the resting group of conductivity maps is at least partially implemented as a forward differential equation solver.

5. The medical imaging system of claim 3, wherein the forward differential equation solver is configured to calculate the conductivity of each voxel using the Laplacian of the B1 phase map for a kernel of voxels surrounding each voxel.

6. The medical imaging system of claim 5, wherein execution of the machine executable instructions further causes the processor to:

   - receive a tissue segmentation assigning each voxel in the region of interest a tissue type;
   - adjust the kernel of voxels surrounding each voxel using the tissue segmentation before calculating the Laplacian, wherein the kernel of voxels surrounding each voxel is adjusted such that all voxels within the kernel have the same tissue type.

7. The medical imaging system of any one of the preceding claims, wherein execution of the machine executable instructions further causes the processor to:

- receive a magnitude image of the region of interest; and
- render the magnitude image and the conductivity change mapping on a display, and wherein any one of the following: the conductivity change mapping is superimposed on the magnitude image and the conductivity change mapping and the magnitude image are displayed in adjacent regions with an identical scale.

8. The medical imaging system of any one of the preceding claim, wherein execution of the machine executable instructions further causes the processor to:

- receive (404) magnetic resonance imaging data (332) acquired according to a B1 phase mapping magnetic resonance imaging protocol descriptive of the region of interest of the subject;
- receive (406) metadata (334) that assigns portions of the magnetic resonance imaging data to a resting state of the subject or an active state of the subject;
- reconstruct (408) multiple B 1 magnetic resonance phase maps of the region of interest of the subject from the portions of the magnetic resonance imaging data;
- construct (410) the active group of B1 phase maps and the resting group of B1 phase maps by assigning each of the multiple B1 magnetic resonance phase maps using the metadata.

9. The medical imaging system of claim 8, wherein the medical imaging system further comprises a magnetic resonance imaging system (302) configured for acquiring the magnetic resonance imaging data from the subject from an imaging zone (308), wherein the memory further comprises pulse sequence commands (330), wherein the pulse sequence commands are configured to control the magnetic resonance imaging system to acquire the magnetic resonance imaging data from the region of interest according to the B1 phase mapping magnetic resonance imaging protocol, wherein the region of interest is within the imaging zone, wherein execution of the machine executable instructions further cause the processor to control the magnetic resonance imaging system to acquire (400) the magnetic resonance imaging data using the pulse sequence commands.

10. The medical imaging system of claim 9, wherein the B1 phase mapping magnetic resonance imaging protocol is any one of the following: a balanced steady-state free precession magnetic resonance imaging protocol, a multi-echo-gradient echo magnetic resonance imaging protocol, and a spin echo based magnetic resonance imaging protocol.

11. The medical imaging system of claim 9 or 10, wherein the magnetic resonance imaging system further comprises a subject indicator configured for indicating the resting state and the active state to the subject, wherein execution of the machine executable instructions further causes the processor to:

- control (400) the magnetic resonance imaging system to repeatedly acquire the magnetic resonance imaging data while the subject indicator alternates between the resting state and the active state; and
- generate (402) the metadata for the magnetic resonance imaging data to match the subject indicator during the acquisition of the magnetic resonance imaging data.

12. The medical imaging system of any one of the preceding claims, wherein the resting group of B1 phase maps and the active group of B1 phase maps each contain any one of the following: at least 5 B1 phase maps each, at least 10 B1 phase maps each, at least 20 B1 phase maps each, at least 40 B1 phase maps each, at least 60 B1 phase maps each, and at least 80 B1 phase maps each.

13. A method of operating a medical imaging system (100, 300), wherein the method comprises:

- receiving (200) a resting group of B1 phase maps (122) of a region of interest (309) of a subject (308);
- receiving (202) an active group of B1 phase maps (124) of the region of interest of the subject;
- calculating (204) a resting group of conductivity maps (126) for the region of interest using the resting group of B1 phase maps according to an electrical properties tomography algorithm;
- calculating (206) an active group of conductivity maps (128) for the region of interest using the active group of B1 phase maps according to the electrical properties tomography algorithm; and
- calculating (208) a conductivity change mapping (130) for the region of interest using the resting group of conductivity maps and the active group of conductivity maps.

**14.** A computer program product comprising machine executable instructions (120) for execution by a processor (104) controlling a medical imaging system (100, 300), wherein execution of the machine executable instructions causes the processor to:

- receive (200) a resting group of B1 phase maps (122) of a region of interest (309) of a subject (318);
- receive (202) an active group of B1 phase maps (124) of the region of interest of the subject;
- calculate (204) a resting group of conductivity maps (126) for the region of interest using the resting group of B1 phase maps according to an electrical properties tomography algorithm;
- calculate (206) an active group of conductivity maps (128) for the region of interest using the active group of B1 phase maps according to the electrical properties tomography algorithm; and
- calculate (208) a conductivity change mapping (130) for the region of interest using the resting group of conductivity maps and the active group of conductivity maps.

Fig. 1

Fig. 2

Fig. 3

Fig. 4

Fig. 5

Fig. 6

Europäisches
Patentamt

European
Patent Office

Office européen
des brevets

# EUROPEAN SEARCH REPORT

Application Number

EP 18 17 6929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | ULRICH KATSCHER ET AL: "Electric properties tomography: Biochemical, physical and technical background, evaluation and clinical applications", NMR IN BIOMEDICINE., vol. 30, no. 8, 24 May 2017 (2017-05-24), page e3729, XP055508183, GB ISSN: 0952-3480, DOI: 10.1002/nbm.3729 * the whole document * * 4.2 Denoising filters; page 6 * | 1-14 | INV. A61B5/00 A61B5/04 |
| Y | JIAEN LIU ET AL: "Gradient-based electrical properties tomography (gEPT): A robust method for mapping electrical properties of biological tissues in vivo using magnetic resonance imaging : Gradient-Based Electrical Properties Tomography", MAGNETIC RESONANCE IN MEDICINE., vol. 74, no. 3, 1 September 2015 (2015-09-01), pages 634-646, XP055512144, US ISSN: 0740-3194, DOI: 10.1002/mrm.25434 * the whole document * * abstract * * In Vivo Human Experiments; page 641 - page 642 * | 1-14 | |
| | -/-- | | TECHNICAL FIELDS SEARCHED (IPC) A61B |

The present search report has been drawn up for all claims

| Place of search | Date of completion of the search | Examiner |
|---|---|---|
| The Hague | 30 November 2018 | Weiss-Schaber, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 2

Europäisches
Patentamt

European
Patent Office

Office européen
des brevets

## EUROPEAN SEARCH REPORT

Application Number

EP 18 17 6929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | J. D. ALLISON ET AL: "Functional MRI cerebral activation and deactivation during finger movement", NEUROLOGY, vol. 54, no. 1, 11 January 2000 (2000-01-11), pages 135-142, XP055512147, US ISSN: 0028-3878, DOI: 10.1212/WNL.54.1.135 * the whole document * * page 136 * ----- | 1-14 | |

TECHNICAL FIELDS
SEARCHED       (IPC)

The present search report has been drawn up for all claims

| Place of search | Date of completion of the search | Examiner |
|---|---|---|
| The Hague | 30 November 2018 | Weiss-Schaber, C |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

# EP 3 581 090 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KATSCHER ; VAN DEN BERG.** Electric properties tomography: Biochemical, physical and technical background, evaluation and clinical applications. *NMR in Biomedicine,* 2017, 2729